# EUROPEAN PATENT SPECIFICATION

(11) **EP 1 572 201 B1**
(45) Date of publication and mention of the grant of the patent: **09.07.2008**
(21) Application number: 03782431.5
(22) Date of filing: 17.12.2003
(51) Int. Cl.: A61K 31/4178, A61K 9/06, A61P 31/10

(54) **PHARMACEUTICAL COMPOSITIONS OF SERTACONAZOLE FOR VAGINAL USE**
PHARMAZEUTISCHE ZUSAMMENSETZUNGEN MIT SERTACONAZOL FÜR VAGINALE VERWENDUNG
COMPOSITIONS PHARMACEUTIQUES DE SERTACONAZOLE A USAGE VAGINAL

(30) Priority: 18.12.2002 WO PCT/EP02/14488
(43) Date of publication of application: 14.09.2005
(73) Proprietor: FERRER INTERNACIONAL, S.A., 08028 Barcelona (ES)
(72) Inventor: PALACIN, Celia, E-08017 Barcelona (ES); GUERRERO, Marta, E-08024 Barcelona (ES); RAGA, Manuel, M., E-08024 Barcelona (ES); ROMERO, Alfonso, E-08012 Barcelona (ES); GUGLIETTA, Antonio, E-08970 Sant Joan Despi (ES)
(74) Representative: Reitstötter - Kinzebach
(86) International application number: PCT/EP2003/014422
(87) International publication number: WO 2004/054576

(56) References cited:
- WO-A-00/30626
- WO-A-99/13862
- WO-A-03/032948
- J. TORRES ET AL: "Sertaconazole in the treatment of mycoses; from dermatolgy to gynecology" INTERNATIONAL JOURNAL OF GYNECOLOGY AND OBSTETRICS, vol. 71, no. S1, December 2000 (2000-12), pages S3-S20, XP002250812 New York (US)

## Description

### Field of the invention

The present invention relates to compositions of sertaconazole for vaginal use and more specifically to compositions of sertaconazole for vaginal use in the treatment of vulvovaginal candidiasis.

### Background of the invention

Vulvovaginal candidiasis is an inflammatory process that affects the vulva, the vagina or both together, and is caused by a superficial infection of the epithelial cells, especially by the yeast *Candida albicans* and to a lesser extent by other *Candida* spp., such as *C*. *glabrata, C*. *tropicalis, C. parapsilosis, C. guillermondi* and C. krusei. Vulvovaginal candidiasis is characterised by vulvar pruritus, vaginal secretion with or without true vaginitis, leucorrhoea, vulvar erythema, and maceration. As the prevalence of this disease is increasing, the research for and development of new antifungal preparations is fully justified. It is nowadays accepted that oral and intravaginal antifungal drugs are similarly effective in the treatment of uncomplicated vulvovaginal candidiasis. Since it is usually preferable to administer medicines topically rather than orally, especially in pregnant women, local treatment of vulvovaginal candidiasis is consequently recommended and oral drug delivery should be avoided whenever possible.

USP 4551148 describes systems for vaginal delivery consisting of emulsions or suspensions of nystatin with characteristics of bioadherence to the vaginal surface. On the other hand, US Patent 5266329 describes systems for vaginal delivery consisting of emulsions or suspensions of imidazole antifungal agents with characteristics of bioadherence to the vaginal surface.

WO95/31178 describes emulsions and aqueous solutions of itraconazole with cyclodextrin for vaginal use.

USP 5514698 describes long-lasting antifungal vaginal creams that have a stable viscosity in the human body.

EP 770384 describes anhydrous solid pharmaceutical compositions of antimycotic agents, antiprotozoal agents, disinfectants, hormones, antibiotics and chemotherapeutic agents for vaginal use containing polycarbophil as a unique mucoadhesive polymer. Similarly, EP 918510 describes gels of polycarbophil-azole complexes with antifungal, or antiprotozoal activity, in which the polycarbophil acts as a mucoadhesive polymer.

WO00/30626 describes a method for treating vulvovaginal candidiasis consisting of the intravaginal administration of a single dose of an ovule of miconazole nitrate as well as the application of miconazole nitrate cream to the vulva.

WO02/03896 describes a method for treating vaginal or uterine infections caused by fungi, bacteria, viruses or parasites that consists of bringing the vaginal epithelium into contact with an intravaginal device that contains an antifungal agent, an antibacterial agent, an antiviral agent or a trichomonocidal agent, including a lipophilic or hydrophilic excipient, a mucoadhesive agent and a penetration enhancer of the active ingredient.

DE-A-19737348 describes synergistic combinations of clindamycin and clotrimazole in the form of tablets, pessaries and ovules for local treatment of bacterial and fungal infections of the vagina.

WO 99/55333 describes synergistic combinations of at least two imidazole ingredients for locally combating the microorganisms that cause vulvovaginitis and vaginosis.

WO 03/032948 describes compositions and methods for delivering antibacterial, antifungal and antiviral ointments to the oral, nasal or vaginal cavity. Said compositions may contain one or more bioadhesive agents, such as xanthan gum and sodium carboxymethylcellulose.

WO 99/13862 relates to pharmaceutical compositions comprising a pharmaceutically acceptable bioadhesive carrier for vaginal administration. The bioadhesive carrier is a cross-linked polycarboxylic acid polymer formulation. Suitable cross-linking agents include divinyl glycol, divinylbenzene, N,N-dialkylacrylamide, 3,4-hydroxy-1,5-hexadiene, 2,5-dimethyl-1,5-hexadiene and similar agents. Suitable polycarboxylic acids include polyacrylic and polymethacrylic acids.

In Torres et al., International Journal of Gynecology & Obstetrics 71 (2000) 53-520 the use of sertaconazole in gynecology is described. The formulations and doses tested were 300 mg sustained release vaginal ovule, 500-mg vaginal tablet (both single dose) and 2 % vaginal cream in repeated applications for 7 days.

### Detailed description of the invention

The object of the present invention is to provide new compositions of sertaconazole for vaginal use in the treatment of vulvovaginal candidiasis. In one aspect, the present invention relates to mucoadhesive vaginal compositions for single dose administration, comprising sertaconazole or one of its pharmaceutically acceptable salts wherein the proportion of sertaconazole or the salt is from 6 to 10% (single-dose dosage forms). In another aspect, the present invention relates to the use of said compositions for the manufacture of a pharmaceutically acceptable dosage form for the treatment of vulvovaginal candidiasis.

No composition of sertaconazole with the aforesaid characteristics has been described to date.

Sertaconazole is a broad-spectrum antifungal agent with excellent activity against yeasts, dermatophytes, and opportunistic fungi. In addition to its antifungal efficacy, sertaconazole has a good safety profile, sustained cutaneous retention and low systemic absorption. All these properties make it be an ideal product for topical application. For reference, the *in vitro* activities, expressed as minimum inhibitory concentrations (MIC), of sertaconazole, bifonazole and econazole against mostly prevalent *Candida* spp. in vulvovaginal candidiasis are shown in Table 1 (Carrillo-Muñoz A.J. and Torres-Rodriguez J.M., J. Antimicrob. Chemother. 1995: 36, 713-716).

**Table 1**

| Microorganism | Sertaconazole | Bifonazole | Econazole |
|---|---|---|---|
| *C. albicans* (73) | 1.02 | 3.6 | 2.24 |
| *C. tropicalis* (21) | 1.67 | 9.51 | 3.14 |
| *C. glabrata* (16) | 0.78 | 4.09 | 2.39 |
| *C. parapsilosis* (22) | 0.31 | 3.76 | 0.75 |
| *C. krusei* (13) | 0.38 | 2.20 | 0.91 |
| *C. guillermondii* (5) | 0.51 | 3.87 | 1.11 |

Furthermore, sertaconzole is superior to most imidazole antifungal drugs as a fungicide against *C. albicans* (Palacín C., Sacristán A. and Ortiz J.A., Arzneim. Forschung, 1992: 42(I), 711-714; Agut J., Palacín C. and Ortiz J.A., Arzneim. Forschung 1992, 42(I), 721-724).

In contrast to prior-art compositions, the present invention is characterised by the presence of one or more mucoadhesive excipients. These mucoadhesive excipients are preferably selected from cellulose polymers, such as carboxymethylcellulose, hydroxypropyl methylcellulose, methylcellulose and the like, or from polyacrylic acid-derivative polymers, such as carbomers, polycarbophils and the like. The applicants have discovered that, surprisingly, the combination of a polycarbophil and a carbomer enhances the mucoadhesive action of the preparation, but not the absorption of sertaconazole. Consequently, the active ingredient, sertaconazole, remains in the mucosa of the vagina for a period of 3 to 5 days, its absorption (permeation) through the vaginal mucosa being less than 0.1% of the dose. As a result, systemic side effects are negligible. The resulting intravaginal preparation requires just a single-dose application to achieve a convenient and safe eradication of *Candida* spp., which is very advantageous in practice.

The excipients used in the present invention are classified as lipophilic, mucoadhesive and preservative. Among the possible excipients, which are not intended to restrict the scope of the present invention, the following are preferred:
a) Lipophilic excipients: Glyceryl stearates and derivatives, for example, polyethylene glycol stearates, ketostearyl alcohols, polyoxyethylene glycol ethers of n-alcohols (lauryl, cetyl, stearyl and myristyl alcohol), liquid paraffin, lecithin oil, glycerol and the like. The applicants have discovered that the combination of palmitate stearate of ethyleneglycol and polyethylene glycol (Tefose 63), saturated polyglycolised glycerides (Labrafil M2130CS), glyceryl isostearate (isostearic peceol) and liquid paraffin proves very suitable for the implementation of the present invention. As a whole, the lipophilic excipients are present in a total proportion of from 10 to 40%, preferably from 30 to 35%, of the composition.
b) Mucoadhesive excipients: Cellulose polymers selected from sodium carboxymethylcellulose, hydroxypropyl methylcellulose, methylcellulose and the like, gelatin, colloidal anhydrous silica, or polyacrylic acid polymers, such as carbomers and polycarbophils. All these mucoadhesive excipients also possess gel-forming capacity. The applicants have discovered that the combination of polyacrylic acid cross-linked with divinyl glycol (e.g. polycarbophil AA-1) and acrylic acid cross-linked with allyl esters of sucrose or pentaerythritol (e.g. carbopol 974P or carbopol 934P) proves very suitable for the implementation of the present invention. As a whole, the mucoadhesive excipients with gel-forming properties are present in a total proportion between 0.1 and 3%, preferably between 1 and 1.5% of the composition.
c) Preservatives: Parabens, such as methylparaben, butylparaben or propylparaben, benzoic acid, sorbic acid, boric acid and the like. As a whole, the preservatives are present in a total proportion between 0.01 and 0.3%, preferably between 0.1 and 0.2% of the composition.

Optionally, the compositions of the present invention can contain in addition suspending agents and humectants, such as povidone or propylene glycol, and neutralising agents for adjusting the viscosity of the composition, such as sodium hydroxide, triethanolamine (TEA) or ethylenediamine tetraacetic acid (EDTA). Povidone is normally used in concentrations of from 1 to 3% of the composition, preferably 2%. As for propylene glycol, it is normally used from 5 to 10% of the composition, preferably 7%.

Among the possible compositions, the invention relates preferably to creams and gels. For preparation of the compositions of the present invention, sertaconazole can be used as free base or in the form of a pharmaceutically acceptable salt. Among the pharmaceutically acceptable salts, the nitrate is preferred. The compositions may also contain mixtures of the free base with one or more salts as well as mixtures of two or more salts.

In principle, cream formulations can be applied at two different concentrations of sertaconazole. The highest concentration cream is applied inside the vagina and the lowest concentration is applied on the periphery of the infected zone, the vulva. The present invention relates more precisely to the cream for internal application, administered in a single dose. The concentration of sertaconazole nitrate in this cream composition can range from 6 to 10%, and its quantity by volume can range from 4 to 6 ml. This corresponds to a dose of from 240 to 600 mg sertaconazole nitrate. According to one embodiment, the concentration is higher than 6% and, in particular, ranges up to 9%. A concentration of up to 8%, and more preferably from 6 to 7% is preferred. A volume of 5 ml is preferred. This corresponds to a dose of up to 400 mg and more preferably from 300 to 350 mg sertaconazole nitrate. For sertaconazole and sertaconazole salts other than the nitrate the same concentrations apply. Alternatively, the present invention also relates to gels for application inside the vagina, which can be administered in a single dose. The concentration of the active ingredient in the gel formulations is similar to that of the corresponding cream formulations. However, the gels in contrast to the creams, do not necessarily contain lipophilic excipients in their formulation. For proper administration of these formulations (creams and gels), they can be conveniently packed inside an applicator, such as that described in ES 2,133,090, which constitutes one of the objects of the present invention. The size of the crystals of sertaconazole nitrate in the resulting cream should be below 80 µm. Preferably, micronized sertaconazole nitrate having a particle size below 80µm is used.

On the other hand, the conventional cream for vulvar application mentioned in the preceding paragraph has a concentration of sertaconazole nitrate between 1 and 3%, preferably 2%. Ita volume can range from 5 to 15 ml, preferably 10 ml. In the case of the cream or gel formulations for internal application, single or repeated doses can be administered. This cream composition is used for alleviating itching and irritation outside the vagina (in the vulva) in women infected with *Candida* spp. in both the vulva and the vagina, and represents a supplemental vaginal therapy with the concentrated cream or gel formulations, as described in the preceding paragraph.

Thus, a preferred embodiment of the present invention is a kit with the two compositions. The concentrated cream or gel formulation for internal use is conveniently packed in an applicator and prepared for its application in a single dose. The conventional cream for external use is packed in a conventional tube for its application in a single or repeated dose.

The release of sertaconazole nitrate from the two formulations, a concentrated intravaginal cream formulation (Example 1) and a conventional cream formulation, was tested. The formulation of the present invention releases the active ingredient in a slow release profile, in contrast to the conventional cream formulation, which is also used for the treatment of the external area (vulva).

Unless indicated otherwise, concentrations and proportions given as percentage [%] refer to weight and thus mean "% by weight".

The present invention is further illustrated by - but not limited to - the following examples.

### EXAMPLE 1: Preparation of 100 g of cream for intravaginal administration

### Composition

| | |
|---|---|
| Micronised sertaconazole nitrate particle size below 80 µm | 6.00 g |
| Tefose 63¹ | 20.00 g |
| Labrafil M 2130 CS² | 5.00 g |
| Isostearic peceol³ | 2.00 g |
| Paraffin oil | 8.00 g |
| Benzoic acid | 0.10 g |
| Polycarbophil⁴ AA-1 | 1.00 g |
| Carbopol 974 P⁵ | 0.30 g |
| Purified water q.s. for | 100.00 g |

| | |
|---|---|
| ¹Tefose 63: Ethylene glycol and polyethylene glycol palmitate stearate ²Labrafil M 2130 CS: Saturated polyglycol glycerides ³Isoestearic peceol: Glyceryl isostearate ⁴Polycarbophil AA-1: Polyacrylic acid cross-linked with divinyl glycol ⁵Carbopol 974 P: Carbomer. Acrylic acid polymer cross-linked with sucrose and pentaerythritol allyl esters. | |

### Physicochemical properties

Appearance: White, odourless (or slight oily odour), semisolid cream of fluid consistency.
Penetrability: 43.5 ± 5% mm.
Viscosity: 347,000 cps ± 45% (25°C).

### EXAMPLE 2: In vitro dissolution and transdermal permeation tests

The *in vitro* dissolution and transdermal permeation tests of sertaconazole nitrate from the cream formulations of Example 1 were evaluated in comparison with a conventional cream formulation of 2% sertaconazole nitrate.

The composition for 100 g of the conventional cream is as follows:

| | |
|---|---|
| Micronised sertaconazole nitrate with particle size below 80 µm | 2.00 g |
| Tefose 63¹ | 20.00 g |
| Labrafil MS 2230² | 5.00 g |
| Isostearic peceol³ | 2.00 g |
| Paraffin oil | 8.00 g |
| Nipagin⁴ | 0.10 g |
| Sorbic acid | 0.10 g |
| Purified water q.s. for | 100.00 g |

| | |
|---|---|
| ¹Tefose 63: Ethylene glycol and polyethylene glycol palmitate stearate ²Labrafil MS 2230: Saturated (C₁₀-C₁₈) polyoxyethylene glycol and glycol glycerides ³Isoestearic peceol: Glyceryl isostearate ⁴Nipagin: Methyl *p*-hydroxybenzoate. | |

Both tests were carried out with Franz cell-type diffusion systems, with a diffusional area of 2.54 cm². 1 ml of cream was placed in the donor compartment and 11 ml of a suitable receptor medium were placed in the receptor compartment. For the dissolution test, a 0.45-µm Millipore membrane of nylon esters was used, and the receptor medium was made up of a mixture of ethanol-water (1:1). Vaginal epithelium was used as permeation membrane and phosphate buffer solution at pH 7.4 was used as the receptor medium.

The 4-cm² membranes used in the permeation test were formed by reconstituted vaginal epithelial cells (5-day culture) from transformed cells of human vaginal epithelium on polycarbonate support. These cells were obtained from cell lines of vulvar epidermoid carcinomas. The test temperature was 32°C for both cases.

In the light of the physicochemical properties of sertaconazole, it can be assumed that the maximum quantity permeated is about 1% of the quantity located on the membrane. Under such assumption, the maximum quantity of sertaconazole that reaches the receptor compartment is 6.18 g/ml.

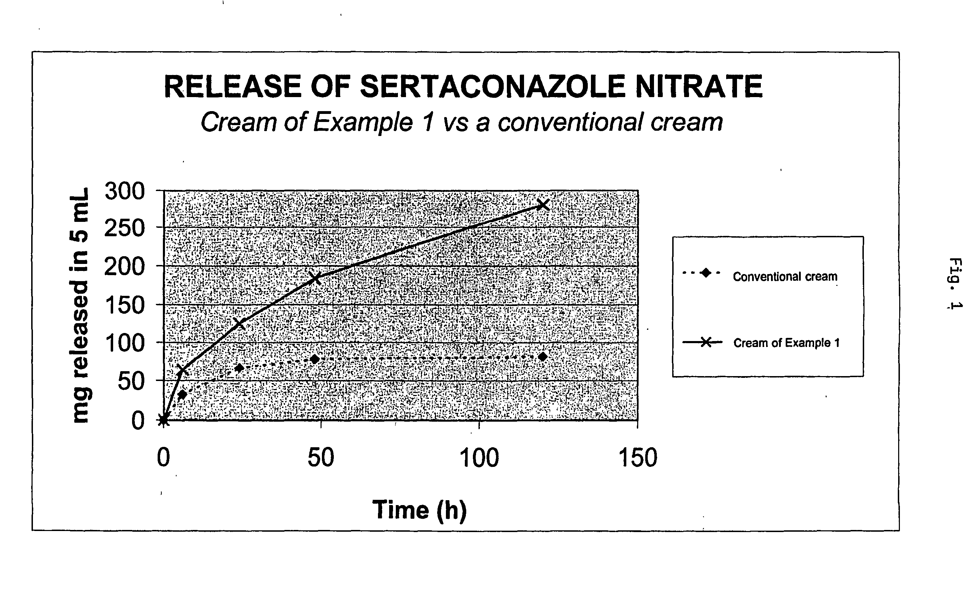

The curves showing the release of sertaconazole nitrate from the two cream formulations are plotted in Fig. 1. Starting from 5 ml of the cream formulation of Example 1, 125 mg of the active ingredient have already been released at 24 hours and on the following days at the rate of 50-60 mg/day. Thus, it is observed that the delivery of the active ingredient is 81% after 5 days.

Moreover, the *in vitro* permeation test shows that the active substance present in the vaginal cream formulation of Example 1 permeates less than 0.1% of the dose.

The toxicity study performed to assess the non-clinical vaginal tolerance of the mucoadhesive cream proves a good tolerance after being applied at single or repeated doses to rats (method according to CMP/SWP/21H5/00).

### EXAMPLE 3: Preparation of 100 g of gel for intravaginal administration

Starting from the appropriate components and according to standard procedures of pharmaceutical technology, the following gel composition was obtained:

| | |
|---|---|
| Micronised sertaconazole nitrate with particle size below 80 µm | 6.00 g |
| Carbopol 974 P¹ | 0.70 g |
| Polycarbophil AA-1² | 0.30 g |
| Propylene glycol | 7.00 g |
| Nipagin³ | 0.16 g |
| Nipasol⁴ | 0.04 g |
| Povidone | 2.00 g |
| TEA⁵ | * |
| Purified water q.s. for | 100.00 g |

| | |
|---|---|
| ¹Carbopol 974 P: Carbomer. Acrylic acid polymer cross-linked with sucrose and pentaerythritol allyl esters. ²Polycarbophil AA-1: Polyacrylic acid cross-linked with divinyl glycol ³Nipagin: Methyl *p*-hydroxybenzoate ⁴Nipasol: Propyl *p*-hydroxybenzoate ⁵TEA: Triethanolamine * Quantity sufficient to adjust the viscosity | |

## Claims

1. A vaginal mucoadhesive composition for single dose administration, comprising sertaconazole or one of its pharmaceutically acceptable salts wherein the proportion of sertaconazole or the salt is from 6 to 10 %.

2. The composition of claim 1, wherein the proportion of sertaconazole or the salt is from 6 to 7 %.

3. The composition of claim 1 or 2, which is a cream or a gel.

4. The composition of any one of claims 1 to 3, wherein the pharmaceutically acceptable salt is sertaconazole nitrate.

5. The composition of any one of claims 3 to 4, wherein the cream contains lipophilic excipients, mucoadhesive excipients and one or more preservatives, and the gel dosage form contains mucoadhesive excipients and one or more preservatives.

6. The composition of claim 5, wherein the lipophilic excipients are selected from glyceryl stearates and their derivatives, ketostearyl alcohols, polyoxyethylene glycol ethers of n-alcohols, liquid paraffin, lecithin oil, glycerol and the like.

7. The composition of claim 6, wherein the lipophilic excipients are present in a total proportion of from 10 to 40%.

8. The composition of claim 7, wherein the lipophilic excipients are present in a total proportion of from 30 to 35%.

9. The composition of claim 5, wherein the mucoadhesive excipients are selected from cellulose polymers, gelatin, colloidal anhydrous silica and polyacrylic acid polymers.

10. The composition of claim 9, wherein the mucoadhesive excipients are polyacrylic acid polymers.

11. The composition of claim 10, wherein the polyacrylic acid polymers form a mixture of polyacrylic acid polymer cross-linked with divinyl glycol and acrylic acid polymer cross-linked with sucrose or pentaerythritol allyl esters.

12. The composition of claim 11, wherein the mixture of polyacrylic acid polymer cross-linked with divinyl glycol and acrylic acid polymer cross-linked with sucrose or pentaerythritol allyl esters are present in a proportion of from 0.1 to 3%.

13. The composition of claim 12, wherein the mixture of polyacrylic acid polymer cross-linked with divinyl glycol and acrylic acid polymer cross-linked with sucrose or pentaerythritol allyl esters are present in a proportion of from 1 to 1.5%.

14. The composition of claim 6, wherein the preservatives are selected from parabens, benzoic acid, sorbic acid, boric acid and the like.

15. The composition of claim 14, wherein the preservatives are present in a total proportion of from 0.01 to 0.3%.

16. The composition of claim 15, wherein the preservatives are present in a total proportion of from 0.1 to 0.2%.

17. The composition of any one of the preceding claims, wherein its content is packed in a single-dose applicator.

18. The composition of claim 17, wherein its capacity is from 4 to 6 ml.

19. The composition of claim 18, wherein its capacity is 5 ml.

20. A kit comprising the composition according to claims 1-19, and a cream composition for vulvar application containing sertaconazole or one of its pharmaceutically acceptable salts.

21. The kit of claim 20, wherein the cream composition for vulvar application contains sertaconazole nitrate.

22. The kit of claim 20, wherein the cream composition for vulvar application contains from 1 to 3% of sertaconazole nitrate.

23. The kit of claim 20, wherein the cream composition for vulvar application contains 2% of sertaconazole nitrate.

24. Use of the composition according to any one of claims 1 to 19 for the manufacture of a pharmaceutically acceptable dosage form for the treatment of vulvovaginal candidiasis of the vagina.

25. The use of claim 24, wherein the composition is for being administered into the vagina of a subject in need of such treatment in a single dose.

26. The use of claim 25, additionally comprising a composition containing sertaconazole or one of its pharmaceutically acceptable salts which is for being applied to the vulva in a single or repeated dose.

## Patentansprüche

1. Vaginale mucoadhäsive Zusammensetzung zur Verabreichung einer Einzeldosis, die Sertaconazol oder eines seiner pharmazeutisch akzeptablen Salze umfasst, wobei der Anteil an Sertaconazol oder dem Salz 6 bis 10 % beträgt.

2. Zusammensetzung nach Anspruch 1, wobei der Anteil an Sertaconazol oder dem Salz 6 bis 7 % beträgt.

3. Zusammensetzung nach Anspruch 1 oder 2, die eine Creme oder ein Gel ist.

4. Zusammensetzung nach einem der Ansprüche 1 bis 3, wobei das pharmazeutisch akzeptable Salz Sertaconazolnitrat ist.

5. Zusammensetzung nach einem der Ansprüche 3 oder 4, wobei die Creme lipophile Exzipienten, mucoadhäsive Exzipienten und ein oder mehrere Konservierungsmittel enthält und die Geldosierungsform mucoadhäsive Exzipienten und ein oder mehrere Konservierungsmittel enthält.

6. Zusammensetzung nach Anspruch 5, wobei die lipophilen Exzipienten ausgewählt sind unter Glycerylstearaten und ihren Derivaten, Cetostearylalkoholen, Polyoxyethylenglycolethern von n-Alkoholen, Flüssigparaffin, Lecithinöl, Glycerin und dergleichen.

7. Zusammensetzung nach Anspruch 6, wobei der Gesamtanteil an lipophilen Exzipienten 10 bis 40 % beträgt.

8. Zusammensetzung nach Anspruch 7, wobei der Gesamtanteil an lipophilen Exzipienten 30 bis 35 % beträgt.

9. Zusammensetzung nach Anspruch 5, wobei die mucoadhäsiven Exzipienten ausgewählt sind unter Cellulosepolymeren, Gelatine, kolloidalem wasserfreiem Siliziumdioxid und Polyacrylsäurepolymeren.

10. Zusammensetzung nach Anspruch 9, wobei die mucoadhäsiven Exzipienten Polyacrylsäurepolymere sind.

11. Zusammensetzung nach Anspruch 10, wobei die Polyacrylsäurepolymere ein Gemisch aus Polyacrylsäurepolymer, mit Divinylglycol quervernetzt, und Acrylsäurepolymer, mit Saccharose oder Pentaerythritolallylestern quervernetzt, bilden.

12. Zusammensetzung nach Anspruch 11, wobei der Anteil an dem Gemisch aus Polyacrylsäurepolymer, mit Divinylglycol quervernetzt, und Acrylsäurepolymer, mit Saccharose oder Pentaerythritolallylestern quervernetzt, 0,1 bis 3 % beträgt.

13. Zusammensetzung nach Anspruch 12, wobei der Anteil an dem Gemisch aus Polyacrylsäurepolymer, mit Divinylglycol quervernetzt, und Acrylsäurepolymer, mit Saccharose oder Pentaerythritolallylestern quervernetzt, 1 bis 1,5 % beträgt.

14. Zusammensetzung nach Anspruch 6, wobei die Konservierungsmittel ausgewählt sind unter Parabenen, Benzoesäure, Sorbinsäure, Borsäure und dergleichen.

15. Zusammensetzung nach Anspruch 14, wobei der Gesamtanteil an Konservierungsmitteln 0,01 bis 0,3 % beträgt.

16. Zusammensetzung nach Anspruch 15, wobei der Gesamtanteil an Konservierungsmitteln 0,1 bis 0,2 % beträgt.

17. Zusammensetzung nach einem der vorhergehenden Ansprüche, wobei ihr Inhalt in einem Einzeldosis-Applikator verpackt ist.

18. Zusammensetzung nach Anspruch 17, wobei ihre Füllmenge 4 bis 6 ml beträgt.

19. Zusammensetzung nach Anspruch 18, wobei ihre Füllmenge 5 ml beträgt.

20. Kit, umfassend die Zusammensetzung nach den Ansprüchen 1 bis 19 und eine Cremezusammensetzung zur Anwendung an der Vulva, die Sertaconazol oder eines seiner pharmazeutisch akzeptablen Salze enthält.

21. Kit nach Anspruch 20, worin die Cremezusammensetzung zur Anwendung an der Vulva Sertaconazolnitrat enthält.

22. Kit nach Anspruch 20, wobei die Cremezusammensetzung zur Anwendung an der Vulva 1 bis 3 % Sertaconazolnitrat enthält.

23. Kit nach Anspruch 20, wobei die Cremezusammensetzung zur Anwendung an der Vulva 2 % Sertaconazolnitrat enthält.

24. Verwendung der Zusammensetzung nach einem der Ansprüche 1 bis 19 zur Herstellung einer pharmazeutisch akzeptablen Dosierungsform zur Behandlung vulvovaginaler Candidose der Vagina.

25. Verwendung nach Anspruch 24, wobei die Zusammensetzung geeignet ist, als Einzeldosis in die Vagina eines Wesens, das einer solchen Behandlung bedarf, verabreicht zu werden.

26. Verwendung nach Anspruch 25, die zusätzlich eine Sertaconazol oder eines seiner pharmazeutisch akzeptablen Salze enthaltende Zusammensetzung umfasst, die geeignet ist, als Einzeldosis oder wiederholte Dosis auf die Vulva aufgetragen zu werden.

## Revendications

1. Une composition mucoadhésive vaginale pour administration en dose unique, comprenant du sertaconazole ou l'un de ses sels pharmaceutiquement acceptables, dans laquelle la proportion de sertaconazole ou du sel est comprise entre 6 et 10%.

2. La composition selon la revendication 1, dans laquelle la proportion de sertaconazole ou du sel est comprise entre 6 et 7%.

3. La composition selon la revendication 1 ou 2, qui est une crème ou un gel.

4. La composition selon l'une quelconque des revendications 1 à 3, **caractérisée en ce que** le sel pharmaceutiquement acceptable est le nitrate de sertaconazole.

5. La composition selon l'une quelconque des revendications 3 ou 4, dans laquelle la crème contient des excipients lipophiles, des excipients mucoadhésifs et un ou plusieurs conservateurs, et la forme galénique du gel comprend des excipients mucoadhésifs et un ou plusieurs conservateurs.

6. La composition selon la revendication 5, dans laquelle les excipients lipophiles sont choisis parmi stéarates de glycérine et leurs dérivés, alcools cétostéaryliques, éthers de polyoxyéthylèneglycol de n-alcools, paraffine liquide, huile de lécithine, glycérol et leurs analogues.

7. La composition selon la revendication 6, dans laquelle la proportion totale des excipients lipophiles est comprise entre 10 et 40%.

8. La composition selon la revendication 7, dans laquelle la proportion totale des excipients lipophiles est comprise entre 30 et 35%.

9. La composition selon de la revendication 5, dans laquelle les excipients mucoadhésifs sont choisis parmi polymères de cellulose, gélatine, silice colloïdale anhydre et polymères d'acide polyacrylique.

10. La composition selon la revendication 9, dans laquelle les excipients mucoadhésifs sont des polymères d'acide polyacrylique.

11. La composition selon la revendication 10, dans laquelle les polymères d'acide polyacrylique forment un mélange de polymère d'acide polyacrylique réticulé avec du divinyl glycol et de polymère d'acide acrylique réticulé avec des esters allyliques de pentaérythritol ou du saccharose.

12. La composition selon la revendication 11, dans laquelle la proportion du mélange de polymère d'acide polyacrylique réticulé avec du divinyl glycol et de polymère d'acide acrylique réticulé avec des esters allyliques de pentaérythritol ou du saccharose est comprise entre 0,1 et 3%.

13. La composition selon la revendication 12, dans laquelle la proportion du mélange de polymère d'acide polyacrylique réticulé avec du divinyl glycol et de polymère d'acide acrylique réticlé avec des esters allyliques de pentaérythritol ou du saccharose est comprise entre 1 et 1,5%.

14. La composition selon la revendication 6, dans laquelle les conservateurs sont choisis parmi parahydroxybenzoates, acide benzoïque, acide sorbique, acide borique et leurs analogues.

15. La composition selon la revendication 14, dans laquelle la proportion totale de conservateurs est comprise entre 0,01 et 0,3%.

16. La composition selon la revendication 15, dans laquelle la proportion totale de conservateurs est comprise entre 0,1 et 0,2%.

17. La composition selon l'une quelconque des revendications précédentes, dont le contenu est conditionné dans un applicateur en dose unique.

18. La composition selon la revendication 17, dont le volume est compris entre 4 et 6 ml.

19. La composition selon la revendication 18, dont le volume est 5 ml.

20. Un kit comprenant la composition selon les revendications 1 à 19, et une crème pour application vulvaire contenant du sertaconazole ou l'un de ses sels pharmaceutiquement acceptables.

21. Le kit selon la revendication 20, dans lequel la composition de crème pour application vulvaire contient du nitrate de sertaconazole.

22. Le kit selon la revendication 20, dans lequel la composition de crème pour application vulvaire contient entre 1 et 3% de nitrate de sertaconazole.

23. Le kit selon la revendication 20, dans lequel la composition de crème pour application vulvaire contient 2% de nitrate de sertaconazole.

24. Utilisation de la composition selon l'une quelconque des revendications 1 à 19 pour la fabrication d'une forme galénique pharmaceutiquement acceptable pour le traitement de la candidose vulvovaginale du vagin.

25. Utilisation selon la revendication 24, dans laquelle la composition est destinée à être administrée dans le vagin d'un sujet ayant besoin de ce traitement en dose unique.

26. Utilisation selon la revendication 25, comprenant en outre une composition contenant du sertaconazole ou l'un de ses sels pharmaceutiquement acceptables et qui est destinée à être appliquée à la vulve en dose unique ou répétée.
